# EUROPEAN PATENT SPECIFICATION

(11) **EP 1 441 770 B1**
(45) Date of publication and mention of the grant of the patent: **29.11.2006**
(21) Application number: 02785326.6
(22) Date of filing: 28.10.2002
(51) Int. Cl.: A61K 47/32, A61K 9/00, A61K 31/167, A61P 15/02

(54) **VAGINAL FORMULATION OF LIDOCAINE FOR TREATING UTERINE DYSRHYTHMIA**
VAGINALE ZUSAMMENSETZUNG ENTHALTEND LIDOCAINE ZUR BEHANDLUNG VON GEBÄRMUTTERRHYTHMUSSTÖRUNG
FORMULATION VAGINALE DE LIDOCAINE POUR TRAITER LA DYSRHYTMIE UTERINE

(30) Priority: 29.10.2001 US 330684 P; 24.10.2002 US 278912
(43) Date of publication of application: 04.08.2004
(73) Proprietor: Columbia Laboratories (Bermuda) Limited, Pembroke HM08 (BM)
(72) Inventor: LEVINE, Howard, L., Oceanside, NY 11572 (US); BOLOGNA, William, J., F-75017 Paris (FR); DE ZIEGLER, Dominique, CH-1293 Geneva (CH)
(74) Representative: Keen, Celia Mary
(86) International application number: PCT/EP2002/012042
(87) International publication number: WO 2003/037381

(56) References cited:
- WO-A-00/10536
- WO-A-91/06290
- WO-A-95/07699
- WO-A-96/10989
- WO-A-98/56323
- WO-A-99/13862
- WO-A-99/15210
- US-A- 4 615 697
- US-A- 5 472 704
- US-A- 5 989 535

## Description

### FIELD OF THE INVENTION

This invention relates to the use of lidocaine and a bioadhesive carrier for treating or preventing uterine dysrhythmia. The invention focuses on local topical use of lidocaine for absorption into local tissue to prevent or treat the underlying abnormal or undesirable muscle contractions that cause pain or discomfort, rather than merely relieving or masking pain or discomfort without affecting the cause.

### BACKGROUND OF THE INVENTION

Pelvic pain may be intermittent or recurrent, or it may be constant and severe, but it is frequently associated with uterine dysrhythmia - abnormal, disordered, or disturbed contractions of the uterus. Pelvic pain is often experienced during menses, as painful menstruation, or dysmenorrhea. Women with chronic pelvic pain associated with menstruation frequently spend one day each month in bed and also may have an additional day each month of reduced activity because of the severity of the pain. Pelvic pain may also be caused by pelvic infections, and diseases of the urinary tract or bowel.

Infertility also may be associated with uterine dysrhythmic conditions, including dysmenorrhea, *See, e.g.* U.S. Patent Application Ser., No. 10/089,796. Uterine dysrhythmias may affect the rapid transport of sperm, thus affecting fertility. Contractility along the female tract (uterus and fallopian tubes) appears to be the primary motor assuring rapid transport of sperm from the cervical area to the distal end of the tubes, where fertilization takes place. Retrograde uterine contractility appears to impede this normal transport mechanism.

Chronic pelvic pain is common in women in the reproductive age group. It causes disability and distress, and results in significant costs to health services. Overall, a woman has about a 5% risk of having chronic pelvic pain for some period of time in her lifetime. In patients with a previous diagnosis of pelvic inflammatory disease this risk is increased fourfold to approximately 20%. Recent epidemiologic data from the United States showed that 14,7% of women in their reproductive ages reported chronic pelvic pain. A total of 15% of these women with chronic pelvic pain reported time lost from work and 45% reported reduced work productivity. In the United States 10% of outpatient gynecologic consultations are for chronic pelvic pain and 40% of laparoscopies are done for chronic pelvic pain.

The pathogenesis of chronic pelvic pain is poorly understood. Often, investigation by laparoscopy may reveal endometriosis, mild to moderate, or it may reveal no obvious cause for pain. There are several possible explanations for chronic pelvic pain including undetected irritable bowel syndrome, the vascular hypothesis where pain is thought to arise from dilated pelvic veins in which blood flow is markedly reduced and altered spinal cord and brain processing of stimuli in women with chronic pelvic pain. As the pathophysiology of chronic pelvic pain is not well understood, its treatment is often unsatisfactory and limited to symptom relief. Currently, the main approaches to treatment include symptomatic treatment of pain with medication, surgery, or possibly psychotherapy and counseling.

Very little is known about effective pharmacologie treatment for chronic pelvic pain, despite the fact that it is a very common chronic pain syndrome. Several different pharmacologic classes of medications have been used to alleviate the symptomatic pain and discomfort, rather than treat or prevent the underlying cause, in patients with chronic pain syndromes: nonsteroidal anti-inflammatory drugs, anticonvulsants, local anesthetics, and opioids. Very lew studies have focused on the actual treatment or prevention of the underlying cause - uterine dyskinetic contractions - in order to treat or prevent chronic pelvic pain.

Dysmenorrhea is associated with pain typically related to the menstrual cycle and can be primary or secondary. Most women experience primary dysmenorrhea at some time during their life. The pain is cramping or sharp and lasts the first few days of the menstrual period. It may radiate to the back, thighs, or deep pelvis. Occasionally, nausea or vomiting occurs. Secondary dysmenorrhea may be due to endometriosis or cervical stenosis or, if associated with heavy menstrual flow, to fibroids, adenomyosis, or large endometrial polyps.

In order to provide local or regional blockade for extended periods, clinicians currently use local anesthetics administered through a catheter or syringe to a site where the pain is to be blocked. This requires repeated administration where the pain is to be blocked over a period of greater than one day, either as a bolus or through an indwelling catheter connected to an infusion pump. These methods have the disadvantage of potentially causing irreversible damage to nerves or surrounding tissues due to fluctuations in concentration and high levels of anesthetic. In addition, anesthetic administered by these methods are generally neither confined to the target area, nor delivered in a linear, continuous manner. In all cases, analgesia rarely lasts for longer than six to twelve hours, more typically four to six hours. In the case of a pump, the infusion lines are difficult to position and secure, the patient has limited, encumbered mobility and, when the patient is a small child or mentally impaired, may accidentally disengage the pump.

U.S. Patent No, 5,700,485 discloses a method and device for administering a local anesthetic combined with a biodegradable polymer incorporated into microspheres. Prolonged release of the anesthetic is obtained by administration with glucocorticoid.

Because high systemic anesthetic concentration can cause irritation or burning to the vagina, as well as other detrimental side effects, there is a need to keep systemic circulation of the anesthesia low. Thus, there is a need for a formulation in which local anesthetics would diffuse preferentially into the cervix for a prolonged period of time to ensure sufficient anesthesia for treating pelvic pain due to dysrhythmic conditions, while keeping systemic circulation low.

Similarly, high systemic levels of other anti-dysrhythmic treating agents may lead to adverse side effects, some of which may be severe. Many classic anti-arrhythmic (and other anti-dysrhythmic) agents themselves have the ability to cause coronary arrhythmia, Other detrimental side effects include without limitation nausea, blurred or yellow vision, precipitation of glaucoma, constipation, seizures, tremor, bone marrow aplasia, pulmonary fibrosis, hypotension, reduction of exercise heart rate, diarrhea and diarrhea-induced hypokalemia, and immunological reactions such as thrombocytopenia, hepatitis, or bone marrow depression. Thus, use of an anti-dyshrythmic agent to treat or prevent uterine dysrhythmia must carefully avoid systemic levels that could prompt coronary problems or other adverse side effects.

Accordingly, there is a need for a new way of reducing or relieving uterine dysrhythmia. The formulation used should avoid blood levels of the treating agent high enough to cause detrimental side effects, while attaining sufficient local tissue levels of the treating agent to provide the desired therapeutic anti-dysrhythmic effect.

### SUMMARY OF THE INVENTION

The invention provides the use of lidocaine and a pharmaceutically acceptable bioadhesive carrier that releases the treating agent over an extended period of time after administration, in the manufacture of a medicament for vaginal administration in reducing or relieving uterine dysthythmia, wherein the lidocaine is present at a concentration of 5% to 12.5% by weight.

### DETAILED DESCRIPTION OF THE INVENTION

The medicament manufactured in accordance with the present invention includes 5 to 12,5% by weight of lidocaine for reducing or relieving uterine dysrhythmia, together with a pharmaceutically acceptable bioadhesive carrier. The lidocaine acts by normalizing propagation of the nerve impulses and/or nerve impulses or cell to cell communication (i.e. faster, slower, or more consistent) causing the abnormal or undesirable contractions.

The bioadhesive carrier includes a bioadhesive, water-swellable, water-insoluble, cross-linked polycarboxylic polymer. In one embodiment the polymer comprise polycarbophil. A preferred carrier, which may be in a gel formulation, contains a polycarbophil base designed to give controlled, extended release of the local anaesthetic through the vaginal mucosa. Similar formulations for administration or different treating agents for other purposes are described in U.S. Patent Nos. 5,543,150 and 6,126,959. In addition, WO 9106290 discloses bioadhesive compositions comprising a bioadhesive polymer such as polycarbophil and various drugs. Vaginal administration is exemplified. WO 9915210 contains a generic disclosure of compositions comprising a mixture of at least two bioadhesive polymers, one of which may be polycarbophil, and various drugs. Vaginal administration is mentioned.

WO 9856323 teaches the treatment of dysmenorrhoea by administering a bioadhesive composition comprising an active agent. WO 9610989 teaches that polycarbophil is a suitable polymer for the controlled administration of drugs (such an anti-STD agents) to the vagina. WO 0010536 also discloses the suitability of polycarbophil for this purpose, as does WO 9507699. However, WO 9507699 specifically mentions progesterone as one of the drugs that may be administered in this way.

U.S. Patent No. 5,543,150 discloses and claims use of similar extended-release vaginal formulations with progesterone to provide a FIRST UTERINE PASS EFFECT: directed, local delivery of the progesterone to effect secretory transformation of the endometrium while maintaining very low blood serum levels of progesterone. Similarly, U.S. Patent No. 6,126,959 discloses and claims use and composition of other similar extended release formulations for vaginal delivery of treating agents to effect local efficacy without also causing detrimental blood levels of the treating agent.

The medicament manufactured in accordance with the invention contains from 5 to 12,5% by weight of lidocaine, typically from 5 to 10% by weight of lidocaine. The medicament is to be applied vaginally, and may be formulated as any appropriate vaginal composition, such as, without limitation, a gel or cream, or even as a gelifying tablet for administration. When administered, the composition diffuses through the vaginal mucosa into the target tissue. Relief from pain is provided by treatment or prevention of the cause or source of the pain, *e.g.* increased or dysrhythmic contractility.

The lidocaine diffuses in high concentrations into the myometrium to alter dysfunctional uterine contractility for control of pain associated therewith. Systemic circulation of the lidocaine remains at a low level, enabling the treatment to avoid adverse systemic side effects. Depending on the formulation, which can be modified to extend or shorten the duration of release of the lidocaine, the release and efficacy of the lidocaine may easily last for at least about 48 hours or more. In one embodiment the medicament is formulated such that a single dosage of about 1 to 1.5g of the medicament will release about 20 to 150 mg of lidocaine over at least about 24 hours after administration. For instance, the medicament may be formulated such that a single dosage will release the treating agent over at least about 48 to 72 hours.

Lidocaine is an antidysrhythmic agent - as are most local anaesthetics. Its chemical formula is 2-(diethylamino)-N-(2,6-dimethylphenyl)acetamide. Its molecular weight is 234.34. Its structural formula is: 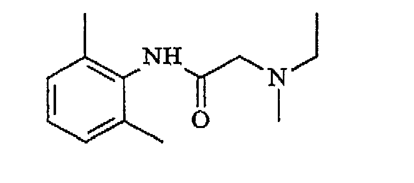 Lidocaine is an extremely safe, effective anaesthetic when it is delivered locally to the site of action - though significant blood serum levels of Lidocaine may also cause adverse side effects. It has a half-life of about 1.5 to 2 hours, which is sufficiently long to make it practical to use in sustained release formulations.

The specific drug delivery formulation chosen includes a cross-linked polycarboxylic acid polymer formulation, generally described in U.S. Patent No. 4,615,697 ("the '697 patent"). In general, at least about 80% of the monomers of the polymer in such a formulation should contain at least one carboxyl functionality. The cross-linking agent should be present at such an amount as to provide enough bioadhesion to allow the system to remain attached to the target epithelial surfaces for a sufficient time to allow the desired dosing to take place. Of course, subject to the limitation that the lidocaine is present at a concentration of 5% to 12.5% by weight, higher doses can be formulated to be released more slowly over a longer period of time; the key factor is the amount of lidocaine administered per unit time, while the concentration of the formulation can be varied inversely with the amount of formulation per unit dosage, or varied directly with the duration of release of the lidocaine. In other words, a higher concentration of treating agent in the formulation can be delivered more slowly, and/or in a smaller dose of the formulation, to achieve the same overall rate of delivery of the treating agent.

For vaginal administration, the formulation preferably remains attached to the epithelial surfaces for a period of about 24 to 48 hours. Such results may be measured clinically over various periods of time, by testing samples from the vagina for pH reduction due to the continued presence of the polymer. This level of bioadhesion is generally attained when the cross-linking agent is present at about 0.1 to 6 weight percent of the polymer, preferably about 1 to 2 weight percent. Bioadhesion can also be measured using commercially available surface tensiometers utilized to measure adhesive strength.

The polymer formulation can be adjusted to control the release rate of the lidocaine, by varying the amount of cross-linking agent in the polymer. Suitable cross-linking agents include divinyl glycol, divinylbenzene, N,N-diallylacrylamide, 3,4-dihydroxy-1,5-hexadiene, 2,5-dimethyl-1,5-hexadiene, and similar agents.

A preferred polymer for use in such a formulation is Polycarbophil, U.S.P., which is commercially available from Noveon, Inc, of Cleveland, Ohio under the trade name NOVEON®-AA1. Polycarbophil is a polyacrylic acid cross-linked with divinyl glycol.

Other useful bioadhesive polymers that may be used in such a drug delivery system formulation are mentioned in the '697 patent. For example, these include polyacrylic acid polymers cross-linked with 3,4-dihydroxy-1,5-hexadiene, and polymethacrylic acid polymers cross-linked with divinyl benzene.

Typically, these polymers would not be used in their salt form, because this would decrease their bioadhesive capability. Divalent salts, such as calcium salts, cause the greatest decrease in bioadhesion. Monovalent salts, such as sodium salts, typically do not reduce bioadhesion as much.

Such bioadhesive polymers may be prepared by conventional free radical polymerization techniques utilizing initiators such as benzoyl peroxide, azobisisobutyronitrile, and the like. Exemplary preparations of useful bioadhesives are provided in the '697 patent.

The bioadhesive formulation may be in the form of a gel, cream, tablet, pill. capsule, suppository, film, or any other pharmaceutically acceptable form that adheres to the mucosa and does not wash away easily. The preferred formulation for the present invention is in the form of a gel.

Additionally, the additives taught in the '697 patent may be mixed in with the cross-linked polymer in the formulation for maximum desired efficacy of the delivery system or for the comfort of the patient. Such additives include, without limitation, one or more of the following: lubricants, plasticizing agents, preservatives, gel formers, tablet, formers, pill formers, suppository formers, film formers, cream formers, disintegrating agents, coatings, binders, vehicles, coloring agents, color controlling against, humectants, viscosity controlling agents, pH-adjusting agents, and other similar commonly-used agents.

The medicament manufactured in accordance with the present invention may be delivered to the vagina in a variety of fashions as known in the art, such as (without limitation) plunger, douche, and manually. One method of delivery is to use a device similar to those described in U.S. Design Patents Nos. D345,211 and D375,352. These devices are oblong hollow tube containers, with one end capable of being opened and the other end containing most of the composition to be delivered in a sealed container that may be used relatively easily by the patient. Said containers also maintain the formulation and treating agent in a sealed, sterile environment unit use. Upon use, such a container is opened and the open end is inserted into the vagina, while the other end is squeezed to deliver the contents of the container into the vagina.

The present invention thus allows the underlying cause of the pain to be treated by delivering sufficient quantity of the lidocaine to the affected tissue for an extended period of time. The delivery system provides a constant source of the drug which achieves concentrations that affect contractility of the tissue, while keeping systemic concentrations low enough to avoid adverse effects.

The lidocaine will generally be used in its basic or unprotonated form. In this form, lidocaine ist only slightly soluble in water. In another from, the lidocaine may be used as water-soluble salts, such as the hydrochloride. The unprotonated form of the lidocaine is necessary for diffusion through cellular membranes to reach the site of action. Cationic species interact preferentially with the Na⁺ channels. In a preferred embodiment, lidocaine is used in its basic form and is suspended in a gel or gelafying tablet for delivery.

Lidocaine, acts on the uterine muscle as an antiarrhythmic and reverse uterine dyskinesia as a means of preventing pain of uterine cramping associated with dyskinesia rather than frequency of contractions. Lidocaine also prevents endometriosis by limiting retrograde menses caused by dysrhythmic contractions, and may also aid sperm transport in women with infertility linked to mild, endometriosis associated with dysmenorrhea.

Typical oral or injection forms of anesthetics such as lidocaine would need to achieve high blood levels in order to reach uterine tissue levels sufficient to demonstrate anti-dysrhythmic efficacy, Even so-called "trigger-points" injections would tend to cause higher blood levels, and present distinct disadvantages with regard to convenience and comfort of administration when compared to the instant formulations.

### EXAMPLES

The following exemplary formulations may be made according to the present invention. All ingredients are listed in percentage by weight.

| | Ex. 1 | Ex. 2 | Ex. 3 | Ex. 4 | Ex. 5 | Ex. 6 |
|---|---|---|---|---|---|---|
| Lidocaine Hydrocloride USP | 6.15 | 6.15 | 6.15 | 6.15 | 6.15 | 6.15 |
| Polycarbophil USP | 1.00 | 0.75 | 1.25 | 1.50 | 1.00 | 0.75 |
| NATROSOL® 250 HHX | 2.00 | 2.25 | 1.50 | 1.50 | 2.00 | 2.00 |
| Glycerol USP/BP | 12.90 | 12.90 | 12.90 | 12.90 | 15.00 | 12.90 |
| Sorbic acid NF/EP | 0.08 | 0.08 | 0.08 | 0.08 | 0.08 | 0.08 |
| Methyl Hydroxybenzoate NF, EP | 0.18 | 0.18 | 0.18 | 0.18 | 0.18 | 0.18 |
| Purified water USP/EP | 77.69 | 77.69 | 77.94 | 77.69 | 75.59 | 77.94 |

| | Ex. 7 | Ex. 8 | Ex. 9 |
|---|---|---|---|
| Lidocaine Hydrocloride USP | 12.30 | 12.30 | 12.30 |
| Polycarbophil USP | 1.00 | 1.00 | 1.00 |
| Carbopol 974P NF | | 1.00 | 1.50 |
| NATROSOL® 250 HHX | 2.00 | 1.00 | |
| Glycerol USP/BP | 12.90 | 12.90 | 12.90 |
| Sorbic acid NF/EP | 0.80 | 0.80 | 0.80 |
| Methyl hydroxybenzoate NF, EP | 0.18 | 0.18 | 0.18 |
| Purified water USP/EP | 70.82 | 70.82 | 71.32 |

A nonlimiting example of a suitable formulation for vaginal delivery of lidocaine comprises polycarbophil, carbopol, NATROSOL®, glycerol, sorbic acid, methyl hydroxybenzoate, and purified water mixed with lidocaine.

Sorbic acid and methylhydroxybenzoate are preservatives, which may be substituted by other known preservatives, such as benzoic acid, propylparaben, or propionic acid.

Carbopol is a gel former, preferably Carbopol 974P, but may be substituted by other gel formers including, but not limited to Carbopol 934P, Carbopol 980, methyl cellulose or propyl cellulose.

NATROSOL® 250 HHX is a viscosity-enhancing agent, but may be substituted by other viscosity-enhancing agents, such as methyl cellulose or propyl cellulose.

Glycerol is a humectant; alternative humectants include, for example, propylene glycol and dipropylene glycol.

As will be apparent to those skilled in the art, the composition can be varied to affect certain properties. For example, the concentration of the bioadhesive polymer can be adjusted to provide greater or lesser bioadhesion. The viscosity can be varied by varying the pH or by changing the concentration of the polymer or gel former. The pH also can be varied as appropriate to affect the release rate or bioadhesiveness of the formulation. All ingredients are well known and readily available from supplier(s) known in the industry. The extended-release formulations enable effective local treatment without also causing blood levels sufficient to induce adverse side effects.

Any and all publications and patent applications mentioned in this specification are indicative of the level of skill of those skilled in the art to which this invention pertains.

## Claims

1. Use of lidocaine and a pharmaceutically acceptable bioadhesive carrier that releases the treating agent over an extended period of time after administration, in the manufacture of a medicament for vaginal administration in reducing or relieving uterine dysrhythmia, wherein the lidocaine is present at a concentration of 5% to 12.5% by weight.

2. Use according to claim 1 wherein the carrier comprises a bioadhesive, water-swellable, water-insoluble, cross-linked polycarboxylic acid polymer.

3. Use according to claim 2, wherein the polymer comprises polycarbophil.

4. Use according to claim 1, wherein the lidocaine is present at a concentration of about 5% to 10% by weight.

5. Use according to claim 4, wherein the medicament is formulated such that a single dosage of about 1 to 1.5 g of the medicament will release about 20 to 150 mg of lidocaine over at least about 24 hours after administration.

6. Use according to claim 5, wherein the medicament is formulated such that a single dosage will release the treating agent over at least about 48 to 72 hours.

## Patentansprüche

1. Verwendung von Lidocain und einer pharmazeutisch annehmbaren bioadhäsiven Trägersubstanz, die den Behandlungswirkstoff während einer ausgedehnten Zeitspanne nach der Verabreichung abgibt, bei der Herstellung eines Medikaments zur vaginalen Verabreichung bei der Reduzierung oder Linderung von Gebärmutterrhythmusstörung, wobei das Lidocain in einer Konzentration von 5 bis 12,5 Gew.-% vorliegt.

2. Verwendung nach Anspruch 1, wobei die Trägersubstanz ein bioadhäsives, im Wasser quellfähiges, wasserunlösliches, quervernetztes Polycarbonsäurepolymer umfasst.

3. Verwendung nach Anspruch 2, wobei das Polymer Polycarbophil umfasst.

4. Verwendung nach Anspruch 1, wobei das Lidocain in einer Konzentration von etwa 5 bis 10 Gew.-% vorliegt.

5. Verwendung nach Anspruch 4, wobei das Medikament so zusammengesetzt ist, dass eine einzelne Dosis von etwa 1 bis 1,5 g des Medikaments etwa 20 bis 150 mg Lidocain während mindestens etwa 24 Stunden nach der Verabreichung abgibt.

6. Verwendung nach Anspruch 5, wobei das Medikament so zusammengesetzt ist, dass eine einzelne Dosis den Behandlungswirkstoff während mindestens etwa 48 bis 72 Stunden abgibt.

## Revendications

1. Utilisation de lidocaïne et d'un véhicule bioadhésif acceptable du point de vue pharmaceutique qui libère l'agent traitant sur une durée prolongée après l'administration, dans la fabrication d'un médicament pour administration vaginale utilisé pour réduire ou soulager la dysrythmie utérine, dans laquelle la lidocaïne est présente à une concentration de 5 % à 12,5 % en poids.

2. Utilisation selon la revendication 1 dans laquelle le véhicule comprend un polymère d'acide polycarboxylique réticulé insoluble dans l'eau, capable de gonfler dans l'eau et bioadhésif.

3. Utilisation selon la revendication 2, dans laquelle le polymère comprend du polycarbophile.

4. Utilisation selon la revendication 1, dans laquelle la lidocaïne est présente à une concentration d'environ 5 % à 10 % en poids.

5. Utilisation selon la revendication 4, dans laquelle le médicament est mis sous forme d'une formulation telle qu'un dosage unique d'environ 1 à 1,5 g du médicament libèrera environ 20 à 150 mg de lidocaïne sur une durée d'au moins environ 24 heures après l'administration.

6. Utilisation selon la revendication 5, dans laquelle le médicament est mis sous forme d'une formulation telle qu'un dosage unique libèrera l'agent traitant sur une durée d'au moins environ 48 à 72 heures.
